(19) Europäisches Patentamt
European Patent Office
Office européen des brevets

(11) **EP 4 524 204 A1**

(12) **EUROPEAN PATENT APPLICATION**

(43) Date of publication:
**19.03.2025 Bulletin 2025/12**

(21) Application number: 24200489.3

(22) Date of filing: **16.09.2024**

(51) International Patent Classification (IPC):
**C08L 93/04** (2006.01)

(52) Cooperative Patent Classification (CPC):
**C08L 93/04**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **18.09.2023 US 202363583329 P**

(71) Applicant: **Kraton Polymers Nederland B.V.
1322 CE Almere (NL)**

(72) Inventors:
• **Eduard, Pieter**
  **Houston, Texas 77084 (US)**
• **den Hartog, Arnold P.**
  **Houston, Texas 77084 (US)**
• **Schaapman, Mark C.**
  **Houston, Texas 77084 (US)**

(74) Representative: **Henkel & Partner mbB
Patentanwaltskanzlei, Rechtsanwaltskanzlei
Maximiliansplatz 21
80333 München (DE)**

(54) **HYDROGENATED DECARBOXYLATED ROSIN ACID**

(57) A hydrogenated decarboxylated rosin acid (HDCR) composition is disclosed comprising, based on total weight of the composition, a plurality of hydrogenated decarboxylated rosin acid (HDCR) components. The HDCR composition contains: (a) 45 to 100 wt.% of the HDCR components have $C_{19}$ carbons and a MW of 262 g/mol; (b) up to 50 wt.% of the HDCR components have $C_{19}$ carbons and a MW of 256 g/mol; (c) up to 20 wt.% of the HDCR components have a MW of 252 g/mol, or $C_{19}$ carbons and a MW of 260 g/mol; and (d) up to 25 wt.% of the HDCR components have $C_{13}$ carbons and a MW of 180 g/mol. The HDCR composition is characterized as having an oxygen content of < 0.5%. The HDCR composition shows enhanced oxidative stability and Gardner color, and can be used in fertilizer coatings, cleaning agents, defoamers, cosmetics, lubricants, etc.

**Description**

**FIELD**

The disclosure relates to a hydrogenated decarboxylated rosin acid (HDCR) composition, methods of preparation, and applications thereof.

**BACKGROUND**

Rosin acid is a valuable resource for various compounds widely used in numerous applications. One modification of rosin acid involves decarboxylation, a process where carboxyl groups are removed, resulting in decarboxylated rosin acid (DCR). This modification is particularly useful for applications where the presence of acidic groups is undesirable.

However, DCR contains unsaturated structures, such as double bonds, which limit its usability in applications that demand high oxidative stability, such as cosmetics, thermal fluids, and medical products. These double bonds make DCR susceptible to oxidation, thereby reducing its stability and effectiveness in these applications.

In the prior art, to enhance the oxidative stability and reduce the color of DCR, the unsaturated structures are converted to saturated ones through hydrogenation. Common hydrogenation processes for DCR typically require severe conditions, including high pressure and high temperature, and the use of specific catalysts. Despite these challenging conditions, there remains some unsaturation in the product, affecting its properties.

There is still a need for a hydrogenated decarboxylated rosin acid (HDCR) composition with improved oxidative stability, increased hydrogenation level, and reduced color, for use to replace fossil-based alternatives, e.g., hydrotreated naphthenic oils, in many applications.

**SUMMARY**

In one aspect, the disclosure relates to a hydrogenated decarboxylated rosin acid (HDCR) composition comprising, consisting essentially of, or consists of: a plurality of hydrogenated decarboxylated rosin acid (HDCR) components. Based on total weight of the composition, the HDCR composition contains: (a) 45 to 100 wt.% of the HDCR components have 19 carbon atoms ($C_{19}$) and a molecular weight of 262 g/mol; (b) up to 50 wt.% of the HDCR components have 19 carbon atoms ($C_{19}$) and a molecular weight of 256 g/mol; (c) up to 20 wt.% of the HDCR components have 19 carbon atoms ($C_{19}$) and a molecular weight of 252 g/mol, or 19 carbon atoms ($C_{19}$) and a molecular weight of 260 g/mol; and (d) up to 25 wt.% of the HDCR components have 13 carbon atoms ($C_{13}$) and a molecular weight of 180 g/mol. The HDCR composition is characterized as having an oxygen content of < 0.5%.

In a second aspect, each HDCR component in the composition has a double bond equivalent (DBE). The HDCR composition has an average DBE of 0.1 to 2, the average DBE is computed as sum of weight percent of each HDCR component multiplied by its DBE.

In a third aspect, the HDCR composition comprises a plurality of hydrogenated decarboxylated rosin acid components having 18 to 20 carbon atoms ($C_{18}$ to $C_{20}$). Each HDCR component in the composition has a double bond equivalent (DBE) and the HDCR composition has: an average DBE of < 2; an oxygen content of < 0.5%; and a Gardner color of < 1. The average DBE is computed as sum of weight percent of each HDCR component multiplied by its DBE.

In a fourth aspect, a method of preparation of a HDCR composition comprises: (m) providing a DCR feedstock; (n) contacting the DCR feedstock with a catalyst, optionally in the presence of a solvent, to obtain a reaction mixture; (o) supplying hydrogen gas with a pressure of 500 to 6000 kPa and heating the reaction mixture at a temperature of 150°C to 350°C; and (p) removing the catalyst and recovering the HDCR composition.

**DESCRIPTION**

The following terms will be used throughout the specification.

"Consisting essentially of" means that the claimed composition primarily contains the specified materials, with allowances for additional components that do not materially affect novel characteristics or function of the claimed invention, with the additional components, if present, in an amount of < 30%, or < 20%, or < 10%.

"At least one of [a group such as A, B, and C]" or "any of [a group such as A, B, and C]," or "selected from [A, B, and C], and combinations thereof' means a single member from the group, more than one member from the group, or a combination of members from the group. For example, at least one of A, B, and C includes, for example, A only, B only, or C only, as well as A and B, A and C, B and C; or A, B, and C, or any other all combinations of A, B, and C. In another example, at least one of A and B means A only, B only, as well as A and B.

A list of embodiments presented as "A, B, or C" is to be interpreted as including the embodiments, A only, B only, C only, "A or B," "A or C," "B or C," or "A, B, or C."

"Any of A, B, or C" refers to one option from A, B, or C.

"Any of A, B, and C" refers to one or more options from A, B, and C.

"Decarboxylated rosin acid" or DCR refers to a compound / material obtained by decarboxylation of rosin acid by removal (or loss) of a carboxyl group (-COOH). The decarboxylation process is typically carried out through heating or other methods, which results in loss of the carboxyl group. This chemical transformation changes the properties of the compound, including its solubility and reactivity.

"Hydrogenation of DCR" refers to a reaction involving the addition of hydrogen ($H_2$) to DCR, resulting in the saturation of carbon-carbon double bonds to obtain a hydrogenated decarboxylated rosin acid (HDCR) composition. The hydrogenation of DCR aims to modify its chemical structure to enhance certain properties or achieve specific characteristics, e.g., stability, color, etc.

"Average Double Bond Equivalent" or "Average DBE" or "Ave. DBE" refers to the degree of unsaturation or the number of double / triple bonds present in a compound, and can be measured by GC-MS. The Ave. DBE is computed based on the DBE of the components in the compound:

$$Ave.\ DBE = sum\ of\ [each\ component\ (wt.\%)\ *\ DBE\ of\ each\ component] \qquad (I)$$

"Hydrogenation percentage (%)" refers to the percentage of double bonds hydrogenated in a compound / molecule relative to the presence of double bonds in the compound / molecule prior to the hydrogenation. Hydrogenation of one double bond corresponds to 100% hydrogenation.

Hydrogenation % of rosin acid is based on the Ave. DBE of rosin acid before and after hydrogenation:

$$Hydrogenation\ \% =$$
$$(Ave.\ DBE\ of\ rosin\ acid) - (Ave.\ DBE\ of\ hydrogenated\ rosin\ acid)\ *\ 100 \qquad (II)$$

Hydrogenation % of HDCR composition is based on the Ave. DBE of DCR feedstock before and HDCR composition after hydrogenation:

$$Hydrogenation\ \% = (Ave.\ DBE\ of\ DCR) - (Ave.\ DBE\ of\ HDCR\ composition)\ *\ 100 \qquad (III)$$

"Oxidative stability" of a compound / material refers to its ability to resist degradation when exposed to oxygen or oxidative conditions at elevated temperatures. Materials with high oxidative stability maintain their chemical integrity and functional properties over time, despite the potential for oxidation. Oxidative stability can be assessed using differential scanning calorimetry (DSC), which measures parameters like the onset of oxidation (T onset) and the maximum rate of oxidative degradation (T max).

Molecular weight (MW) of compounds, components, or species in a compound can be determined by MS (mass spectroscopy), preferably in combination with a chromatographic separation method like GC (gas chromatography) or HPLC (high performance liquid chromatography). In embodiments, the MW is determined by GC-MS, using a column with a highly-substituted cyanopropyl phase (e.g. Supelco SP-2330, Restek Rtx-2330, or Agilent HP-88) of the size 30m × 0.25mm × 0.20μm, with the following operating parameters: a temperature profile of 100°C for 5.0 min, heating with 5°C/min to 250°C and holding this temperature for 10.00 min; forming a solution with 10 mg of compound in 1 ml of a suitable solvent such as toluene, cyclohexane, etc.; injecting 1 μl of the solution with a split ratio of 1:40 at 250°C; maintaining the flow at 1 ml/min throughout the analysis. Identification of the individual components is performed by QMS (quadrupole mass spectrometry) detector, with an ion source temperature of 200°C and a mass range of 35 - 500 amu.

The disclosure relates to a hydrogenated decarboxylated rosin acid (HDCR) composition containing a plurality of HDCR components. The HDCR composition demonstrates enhanced oxidative stability and Gardner color, and finds utility across various applications, e.g., fertilizer coatings, cleaning agents, defoamers, cosmetics, lubricants, etc.

(Hydrogenated Decarboxylated Rosin Acid (HDCR) Composition): The HDCR composition is obtained by hydrogenation of a decarboxylated rosin acid (DCR) feedstock in the presence of a catalyst and at a suitable temperature and pressure. Hydrogenation of the DCR feedstock results in a reduction of C=C double bonds to obtain hydrogenated components.

The DCR feedstock is obtained by decarboxylation of a rosin acid, or by dimerizing and decarboxylation of the rosin acid followed by separation / removal of the dimerized species. The DCR feedstock comprises a plurality of DCR components, e.g., compound-A, compound-B, compound-C, compound-D, etc. In embodiments, the DCR feedstock comprises, based on total weight of the DCR feedstock: (ia) < 30, or 1 - 25, or 3 - 20 wt.% compound-A having 19 carbon atoms ($C_{19}$) and a MW of 262 g/mol; (ib) > 40, or 40 - 60, or 43 - 55 wt.% compound-B having 19 carbon atoms ($C_{19}$) and a MW of 256 g/mol; (ic) > 20, or 10 - 40, or 12 - 35 wt.% of compound-C having 19 carbon atoms ($C_{19}$) and any of a MW of 260 and 252 g/mol; and (id) < 10, or < 5, or 0 - 10 wt.% of compound-D having 13 carbon atoms ($C_{13}$) and a MW of 180 g/mol.

After hydrogenation of the DCR feedstock, the HDCR composition is obtained, comprising a plurality of HDCR components, e.g., compound-I, compound-II, compound-IIIa, compound-IIIb, compound-IV, etc.

In embodiments, compound-I is $C_{19}H_{34}$; compound-II is $C_{19}H_{28}$; compound-IIIa is $C_{19}H_{24}$; compound-IIIb is $C_{19}H_{32}$; and compound-IV is $C_{13}H_{24}$.

In embodiments, the HDCR composition comprises, based on total weight of the HDCR composition: (a) 45 - 100 wt.% of the compound-I having 19 carbon atoms ($C_{19}$) and a MW of 262 g/mol; (b) 0 - 50 wt.% of the compound-II having 19 carbon atoms ($C_{19}$) and a MW of 256 g/mol; (c) 0 to 20 wt.% of the compound-IIIa having 19 carbon atoms ($C_{19}$) and a MW of 252 g/mol or the compound-IIIb having 19 carbon atoms ($C_{19}$) and a MW of 260 g/mol; and (d) 0 to 25 wt.% of the compound-IV having 13 carbon atoms ($C_{13}$) and a MW of 180 g/mol.

In embodiments, the HDCR composition comprises: 50 - 85 wt.% of the compound-I; 0 - 40, or 15 - 40 wt.% of the compound-II; 0 - 15 wt.% of the compound-IIIa or IIIb; and 5 - 15 wt.% of the compound-IV.

The molecular weights of the HDCR components in the HDCR composition can be measured using any of MS, MS / GC / HPLC, and GC-MS. In an embodiment using the GC-MS method and column described above, the HDCR components and their associated MWs can be identified by the following retention profile (with ranges in minutes):

MW of 174 g/mol, 7.0 - 8.5 minutes;
MW of 180 g/mol, 2.5 - 4.0 minutes;
MW of 248 g/mol, 32.5 - 34.5 minutes;
MW of 250 g/mol, 26.0 - 31.0 minutes;
MW of 252 g/mol, 24.5 - 31.0 minutes;
MW of 254 g/mol, 16.5 - 25.0 minutes;
MW of 256 g/mol, 16.5 - 25.0 minutes;
MW of 260 g/mol, 11.0 - 16.0 minutes; and
MW of 262 g/mol (compound-I), 11.0 - 16.0 minutes.

From the GC-MS retention profile above, the various HDCR components can be identified, e.g., compound-IV with a MW of 180 g/mol can be identified with a GC-MS peak in the range of 2.5 - 4.0 min., compound II with a MW of 256 g/mol can be identified in the range of 16.5 - 25.0 min., etc.

In the event of overlapping or identical retention time ranges for different HDCR components, the mass spectrum of each peak (as provided by the GC-MS) can be used to identify the molecular weight of the component, e.g., by comparing the mass spectra of the component with the mass spectra in reference databases like NIST or via library matching.

The species with the same molecular weights (isomers) are clustered and the total amount per isomer is reported.

In embodiments, the HDCR composition comprises several isomers of the compound-I in amounts of > 3, or > 5, or > 10, or > 20, or > 50, or > 100, or < 300.

In embodiments, the HDCR composition comprises C=C double bonds in amounts of < 40%, or < 30%, or < 20%, or < 15%, or < 10%, or < 5%, or > 1%, or 1 - 40%, or 1 - 20%, or 1 - 10%.

In embodiments, the HDCR composition has an average Double Bond Equivalent (DBE) of 0.1 - 2, or 0.2 - 1.5, or 0.5 - 1.4, or 0.5 - 2, or 0.01 - 1, or 0.05 - 0.9, or 0.08 - 0.8, or 0.1 - 0.5, or < 2, or < 1.8, or < 1.5, or < 1.2, or < 1, or < 0.8, or < 0.5, or > 0.01, or > 0.1.

In embodiments, the HDCR composition has a hydrogenation % of > 50%, or > 60%, or > 70%, or > 80%, or > 100%, or > 120%, or > 140%, or > 160%, or > 180%, or > 200%, or > 240%, or < 400%.

In embodiments, the HDCR composition comprises tricyclic compounds having 18 - 20 carbon atoms in amounts of 70 - 100, or 75 - 95, or 75 - 100, or 80 - 100, or 80 - 95, or 80 - 90 wt.%, based on total weight of the HDCR composition.

In embodiments, sum of tricyclic compounds as aromatic and cycloaliphatic in the HDCR composition is at least 70, or > 75, or > 80, or > 85, or > 90, or > 95, or < 99, or 75 - 98, or 80 - 95, or 80 - 98 wt.%, based on total weight of the HDCR composition.

In embodiments, the HDCR composition comprises tricyclic cycloaliphatic compounds in amounts of > 55, or > 60, or > 65, or > 70, or 55 - 90, or 60 - 85, or 60 - 95 wt.%, based on total weight of the HDCR composition.

In embodiments, the HDCR composition comprises components having < 18 carbon atoms in amounts of 0 - 30, or 1 - 20, or 0 - 15, or 1 - 15 wt.%, based on total weight of the HDCR composition.

In embodiments, the HDCR composition comprises $C_{19}H_{20}$ to $C_{19}H_{34}$ species in amounts of 70 - 100, or 75 - 95, or 75 - 100, or 80 - 100, or 80 - 95 wt.%, based on total weight of the HDCR composition.

It should be noted that the feedstock to the hydrogenation process, or the HDCR composition can be prepared from a starting material other than a purified decarboxylated rosin acid stream. In embodiments, the starting material is any of TOR (tall oil rosin) or gum rosin sources, wherein heat (e.g., between 200°C and 300°C) is applied to induce decarboxylation and remove the carboxyl group (-COOH) from the rosin acids, converting them into neutral compounds for a decarboxylated product, e.g., dehydroabietic acid or abietane-type compounds, for the next step of hydrogenation to make the HDCR composition.

(Methods of Preparation of HDCR Composition): As opposed to the prior art severe hydrogenation conditions with high pressure and high temperature and the use of specific catalysts, the HDCR composition in this disclosure is obtained by hydrogenating the DCR to a large extent under relatively mild conditions. In embodiment, the HDCR composition is prepared by hydrogenating the DCR feedstock in the presence of a catalyst, optionally in the presence of a solvent, at a hydrogen gas pressure of < 3000, or < 2500, or 500 - 2000, or 700 - 1800, or 1000 - 1600, or 800 - 1600, or 700 - 1900 kPa; and a temperature of < 350°C, or 150 - 330°C, or 160 - 320°C, or 180 - 300°C, or 250 - 310°C, or 210 - 290°C, or > 180°C, or > 200°C. In embodiments, the reaction is continued for > 1 hr., or > 2 hrs., or < 10 hrs., or 5 - 15 hrs., or 5 - 8 hrs., followed by cooling the reaction mixture at an ambient temperature (e.g., 25°C). At high temperatures, e.g., > 300°C, the hydrogenation can be completed in < 1 hr. After completion of the hydrogenation reaction, catalyst and solvent are removed to recover the HDCR composition.

Examples of solvents (if present) include acids, such as, acetic, propionic, butyric, isobutyric, valeric, etc.; alcohols, such as, methyl, ethyl, propyl, isopropyl, n-butyl, - secondary butyl, amyl, cyclohexyl alcohol, etc.; ethers, such as, dimethyl ether, ethyl methyl ether, diethyl ether, dipropyl ether, diisopropyl ether, ethyl propyl ether, etc.; esters, such as, methyl acetate, ethyl acetate, ethyl propionate, isopropyl acetate, methyl propionate, isopropyl propionate, etc.; hydrocarbons, such as, 2,2,3,3 tetramethyl butane, neo-pentane, 2,2,3,3,4,4 hexamethyl pentane, 2,3 dimethyl butane, etc.

In embodiments, the catalyst is selected from the group consisting of palladium, rhodium, ruthenium, platinum, osmium, nickel, molybdenum, cobalt, iron, and oxides and mixtures thereof. The catalyst can be supported on a support, e.g., diatomaceous earth, carbon, alumina, silica, titania, etc. The amount of catalyst used depends on the hydrogenation rate, the reaction time, the specifications of the reaction vessel, etc. In embodiments, the catalyst is used in amounts of 0.001 - 20, or 0.01 - 15, or 0.05 - 10, or 0.01 - 10, or 0.01 - 5 wt.%, based on total weight of all reactants.

In embodiments, the hydrogen pressure is increased mid-way in the hydrogenation process to speed up the hydrogenation reaction, and / or increasing the hydrogenation rate, e.g., up to 6000, or > 2000, or > 2500, or > 3000, or > 4000, or > 4500 kPa.

In embodiments, further hydrogenation of the HDCR composition is conducted in the same reaction conditions as described above and using the same or different catalyst to further reduce remaining C=C double bonds, for an average DBE of < 1, or < 0.5, or < 0.25, or 0.05 - 0.75.

(Properties of HDCR Composition): The HDCR composition is substantially free of non-aromatic unsaturation or C=C double bonds. With the reduction of unsaturation, the HDCR composition is characterized as having improved oxidative stability, light colored (e.g., water white), and with a low sulfur content.

In embodiments, the HDCR composition has an oxygen content of < 0.5%, or < 0.4%, or < 0.3%, or < 0.2%, or < 0.1%, or 0 - 0.5%, or 0 - 0.3%, or 0 - 0.1%. The oxygent content can be calculated as the oxygen to carbon ratio, or the sum of oxygen atoms present divided by sum of carbon atoms present in the HDCR composition. The number of oxygen and carbon atoms can be obtained from elemental analyses.

In embodiments, the HDCR composition has an acid value of < 10, or < 8, or < 5, or < 2, or < 1, or < 0.8, or < 0.5, or < 0.2, or 0.01 - 10, or 0.1 - 5, or 0.01 - 2 mg KOH/g, measured according to ASTM D1240 or ASTM D465.

In embodiments, the HDCR composition has a viscosity at 40°C of 5 - 50, or 10 - 45, or 15 - 40, or 10 - 30 cSt, measured according to ASTM D-445.

In embodiments, the HDCR composition has an aniline point of 3 - 80°C, or 5 - 70°C, or 5 - 60°C, or 5 - 50°C, or < 80°C, or < 70°C, or < 60°C, measured according to ASTM D611.

In embodiments, the HDCR composition has a pour point of -40 to -10°C, or - 35 to -20°C, or -35 to -25°C, measured according to ASTM D97.

In embodiments, the HDCR composition has a flash point of 95 - 140°C, or 100 - 135°C, or 95 - 135°C, or > 95°C, or > 100°C, or < 140°C measured according to ASTM D92.

In embodiments, the HDCR composition has a Gardner Color of < 1, or < 0.8, or < 0.5, or < 0.2, or 0 - 0.5, or 0.1 - 1, or 0.15 - 0.8, or 0.1 - 0.5, measured according to ASTM D6166.

In embodiments, the HDCR composition has a sulfur content of < 10 ppm, or < 8 ppm, or < 5 ppm, or > 0.001 ppm, measured according to ASTM D5453.

In embodiments, the HDCR composition has a Tonset of > 168°C, or > 170°C, or > 175°C, or > 180°C, or > 185°C, or > 190°C, or > 192°C, or < 200°C, or < 210°C, or 168 - 210°C.

In embodiments, the HDCR composition has a T max of > 183°C, or > 185°C, or > 190°C, or > 195°C, or > 200°C, or > 205°C, or > 210°C, or < 220°C, or 183 - 220°C.

(Applications of HDCR Compositions): The HDCR composition can be used in applications including adhesives, flexographic printing, polymer compatibilizer, tackifier, plasticizer, reinforcing agent, extender in bitumen and asphalt, tires, oilfield and gas industries, carpet construction, road marking, fertilizer coating, cleaning solvent, defoamer, metal working fluid, cosmetics, heat transfer fluid, waterproofing, alkali-, acid- and moisture resistance of sealants, emulsifier for emulsion polymerization, etc.

In embodiments, the HDCR composition is used to modify hydrocarbon products to increase naphthenic and cyclic contents, to improve additive solubility and increase dosage.

(Examples): The following illustrative examples are non-limiting.

The components used in examples include:

DCR-1 is a decarboxylated rosin acid having an acid value of 2.5 mg KOH/g, sulfur content of 81 ppm, Gardner color of 2.3, and oxygen content of 0.1%.

DCR-2 is a decarboxylated rosin acid having an acid value of 0.7 mg KOH/g, sulfur content of < 5 ppm, Gardner color of 2.9, and oxygen content of 0%.

Rosin acid-1 (RA-1) has an acid value of 160 mg KOH/g, sulfur content of 29 ppm, Gardner color of 8.3, and oxygen content of 8.7%.

Rosin acid-2 (RA-2) has an acid value of 183 mg KOH/g, sulfur content of 322 ppm, Gardner color of 4.4, and oxygen content of 10%.

Rosin acid-3 (RA-3) has an acid value of 159 mg KOH/g, sulfur content of < 10 ppm, Gardner color of 11.7, and oxygen content of 8.3%.

(Example 1): 1200 gm of DCR-1 feedstock was charged in a reactor and added 12 gm of Ni catalyst. Reaction content was mixed and purged with nitrogen gas three times at 350 kPa pressure, and then flushed with hydrogen gas three times at 1000 kPa pressure. At this stage, 1000 kPa of hydrogen gas was added to the reactor and reaction content was heated to 270°C followed by increasing the pressure of hydrogen gas to 4000 kPa. Reaction was continued for 10 hours followed by cooling at an ambient temperature (e.g., 25°C). Reaction content was filtered to remove the catalyst and HDCR-1 composition was recovered. A similar procedure was used to hydrogenate aged DCR-1 feedstock (1 year aging) and obtained HDCR-1a composition.

(Example 2): Procedure of example 1 was repeated to use DCR-2 feedstock in place of DCR-1 feedstock.

(Examples 3 - 5): Procedure of example 1 was repeated to use rosin acid feedstocks (e.g., RA-1, RA-2, RA-3, etc.) in place of DCR-1 feedstock.

(Example 6): Procedure of example 1 was repeated to use HDCR-1a composition in place of DCR-1 feedstock and palladium catalyst was used to further hydrogenate HDCR-1b composition.

Table 1 presents details of DCR and RA feedstocks used in hydrogenation reaction and their corresponding hydrogenated compositions. Table 2 shows properties of DCR and RA feedstocks, and their corresponding hydrogenated compositions. Table 3 presents oxidative stability of DCR-1 feedstock and corresponding hydrogenated compositions.

Table 1

| Species with number of carbons | Molecular weight (g/mol) | DBE | DCR-1 | Ex-1 HDCR-1 | Ex-1a HDCR-1a | DCR-2 | Ex-2 HDCR-2 | RA-1 | Ex-3 HRA-1 | RA-2 | Ex-4 HRA-2 | RA-3 | Ex-5 HRA-3 | Ex-6 HDCR-1b |
|---|---|---|---|---|---|---|---|---|---|---|---|---|---|---|
| C13 | 180 | 0 | 0 | 8 | 10 | 0 | 11 | 0 | 0 | 0 | 0 | 0 | 0 | 9 |
| C13 | 174 | 3 | 8 | 0 | 0 | 12 | 0 | 0 | 0 | 0 | 0 | 0 | 0 | 0 |
| C19 | 262 | 0 | 9 | 61 | 49 | 10 | 59 | 0 | 0 | 0 | 0 | 0 | 0 | 81 |
| C19 | 260 | 1 | 13 | 0 | 0 | 19 | 0 | 0 | 4 | 0 | 31 | 0 | 7 | 0 |
| C19 | 256 | 3 | 49 | 26 | 36 | 45 | 27 | 1 | 3 | 0 | 2 | 0 | 1 | 8 |
| C19 | 254 | 4 | 1 | 0 | 0 | 1 | 0 | 0 | 0 | 0 | 3 | 0 | 0 | 0 |
| C19 | 252 | 5 | 13 | 0 | 0 | 10 | 0 | 0 | 0 | 0 | 0 | 0 | 0 | 0 |
| C19 | 250 | 6 | 1 | 0 | 0 | 0 | 0 | 0 | 0 | 0 | 0 | 0 | 0 | 0 |
| C19 | 248 | 7 | 1 | 0 | 0 | 0 | 0 | 0 | 0 | 0 | 0 | 0 | 0 | 0 |
| Saturated fatty acids | - | 0 | 0 | 0 | 0 | 0 | 0 | 0 | 0 | 1 | 2 | 2 | 0 | 0 |
| Tetrahydroabietic acid | - | 0 | 0 | 0 | 0 | 0 | 0 | 0 | 13 | 0 | 0 | 0 | 25 | 0 |
| Mono-unsaturated rosin acids | - | 1 | 0 | 0 | 0 | 0 | 0 | 21 | 9 | 2 | 18 | 0 | 21 | 0 |
| Di-unsaturated rosin acids | - | 2 | 0 | 0 | 0 | 0 | 0 | 6 | 0 | 76 | 0 | 81 | 0 | 0 |
| Dehydroabietic acid | - | 3 | 0 | 0 | 0 | 0 | 0 | 50 | 54 | 18 | 40 | 5 | 34 | 0 |
| Dehydro-dehydroabietic acid | - | 4 | 0 | 0 | 0 | 0 | 0 | 1 | 0 | 1 | 0 | 1 | 0 | 0 |
| (dehydrogenated dehydroabietic acid) | | | | | | | | | | | | | | |
| Unidentified (other) | - | - | 5 | 5 | 5 | 3 | 3 | 21 | 17 | 2 | 4 | 11 | 12 | 2 |
| Average DBE | - | - | 2.66 | 0.78 | 1.08 | 2.44 | 0.81 | 1.9 | 1.84 | 2.12 | 1.87 | 1.81 | 1.33 | 0.24 |
| Total: | - | - | 100 | 100 | 100 | 100 | 100 | 100 | 100 | 100 | 100 | 100 | 100 | 100 |

Table 2

| | Ave. DBE | Hydrogenation (%) | Acid value (mg KOH/g) | Sulfur content (ppm) | Gardner color | Softening point (°C) | Oxygen content (%) |
|---|---|---|---|---|---|---|---|
| DCR-1 | 2.7 | - | 2.5 | 81 | 2.3 | - | 0.1 |
| Ex-1 HDCR-1 | 0.8 | 188 | < 0.1 | <5 | 0 | - | 0 |
| Ex-1a HDCR-1a | 1.1 | 158 | 0.2 | < 5 | 0 | - | 0 |
| DCR-2 | 2.4 | - | 0.7 | <5 | 2.9 | - | 0 |
| Ex-2 HDCR-2 | 0.8 | 163 | 0.2 | < 5 | 0 | - | 0 |
| RA-1 | 1.9 | - | 160 | 29 | 8.3 | 81 | 8.7 |
| Ex-3 HRA-1 | 1.9 | 6 | 145 | < 10 | 7.9 | 67 | 7.9 |

(continued)

| | Ave. DBE | Hydrogenation (%) | Acid value (mg KOH/g) | Sulfur content (ppm) | Gardner color | Softening point (°C) | Oxygen content (%) |
|---|---|---|---|---|---|---|---|
| RA-2 | 2.1 | - | 183 | 322 | 4.4 | 69 | 10 |
| Ex-4 HRA-2 | 1.9 | 25 | 107 | < 10 | 5.6 | - | 5.8 |
| RA-3 | 1.8 | - | 159 | < 10 | 11.7 | 79 | 8.7 |
| Ex-5 HRA-3 | 1.3 | 48 | 152 | < 10 | 5.9 | 66 | 8.3 |
| Ex-6 HDCR-1b | 0.2 | 84 | < 0.1 | <5 | 0 | - | 0 |

Table 3

| | T onset (°C) | T max (°C) |
|---|---|---|
| DCR-1 | 168 | 183 |
| Ex-1 HDCR-1 | 188 | 206 |
| Ex-6 HDCR-1b | 194 | 211 |

As used herein, the term "comprising" means including elements or steps that are identified following that term, but any such elements or steps are not exhaustive, and an embodiment can include other elements or steps. Although the terms "comprising" and "including" have been used herein to describe various aspects, the terms "consisting essentially of" and "consisting of" can be used in place of "comprising" and "including" to provide for more specific aspects of the disclosure and are also disclosed.

## Claims

1. A hydrogenated decarboxylated rosin acid composition comprising, based on total weight of the composition, a plurality of hydrogenated decarboxylated rosin acid (HDCR) components, wherein:
   45 to 100 wt.% of the HDCR components have 19 carbon atoms ($C_{19}$) and a molecular weight of 262 g/mol;

   up to 50 wt.% of the HDCR components have 19 carbon atoms ($C_{19}$) and a molecular weight of 256 g/mol;
   up to 20 wt.% of the HDCR components have 19 carbon atoms ($C_{19}$) and a molecular weight of 252 g/mol, or 19 carbon atoms ($C_{19}$) and a molecular weight of 260 g/mol; and
   up to 25 wt.% of the HDCR components have 13 carbon atoms ($C_{13}$) and a molecular weight of 180 g/mol;

   wherein the hydrogenated decarboxylated rosin acid composition has an oxygen content of < 0.5%, measured as described in the specification.

2. A hydrogenated decarboxylated rosin acid composition comprising a plurality of hydrogenated decarboxylated rosin acid (HDCR) components having 18 to 20 carbon atoms ($C_{18}$ to $C_{20}$); wherein
   each HDCR component in the composition has a double bond equivalent (DBE); wherein the hydrogenated decarboxylated rosin acid composition has:

   an average DBE of < 2;
   an oxygen content of < 0.5%; and
   a Gardner color of < 1, measured according to ASTM D6166;

   wherein the average DBE is computed as sum of weight percent of each HDCR component multiplied by its DBE.

3. The hydrogenated decarboxylated rosin acid composition of claim 2, wherein the hydrogenated decarboxylated rosin acid composition has an average DBE of 0.1 to 2.

4. The hydrogenated decarboxylated rosin acid composition of any of claims 1-3, wherein the hydrogenated decarboxylated rosin acid composition has a Gardner color of < 0.5.

5. The hydrogenated decarboxylated rosin acid composition of any of claims 1-4, wherein the hydrogenated decarboxylated rosin acid composition has a hydrogenation percentage of > 100%, measured as described in the specification.

6. The hydrogenated decarboxylated rosin acid composition of any of claims 1-5, wherein the HDCR components having 19 carbon atoms ($C_{19}$) and a molecular weight of 262 g/mol have at least 3 isomers.

7. The hydrogenated decarboxylated rosin acid composition of any of claims 1-6, wherein 80 to 95 wt.% of the HDCR components are tricyclic compounds with 18 to 20 carbon atoms.

8. The hydrogenated decarboxylated rosin acid composition of claim 7, wherein at least 70 wt.% of the tricyclic compounds with 18 to 20 carbon atoms are aromatic and cycloaliphatic.

9. The hydrogenated decarboxylated rosin acid composition of claim 7, wherein at least 55 wt.% of the tricyclic compounds that are aromatic and cycloaliphatic are cycloaliphatic compounds.

10. The hydrogenated decarboxylated rosin acid composition of any of claims 1-9, wherein the hydrogenated decarboxylated rosin acid composition has an oxygen content of < 0.3%.

11. The hydrogenated decarboxylated rosin acid composition of any of claims 1-10, wherein the hydrogenated decarboxylated rosin acid composition comprises, based on total weight of the composition:

   50 to 85 wt.% of the components having 19 carbon atoms ($C_{19}$) and a molecular weight of 262 g/mol;
   15 to 40 wt.% of the components having 19 carbon atoms ($C_{19}$) and a molecular weight of 256 g/mol;
   up to 15 wt.% of the components having 19 carbon atoms ($C_{19}$) and a molecular weight of 252 g/mol, or 19 carbon atoms ($C_{19}$) and a molecular weight of 260 g/mol; and
   5 to 15 wt.% of the components having 13 carbon atoms ($C_{13}$) and a molecular weight of 180 g/mol.

12. The hydrogenated decarboxylated rosin acid composition of any of claims 1-11, wherein the hydrogenated decarboxylated rosin acid composition has at least one of:

   an acid value of < 10 mg KOH/g, measured according to ASTM D1240;
   a Gardner color of 0.1 to 1; and
   a sulfur content of < 10 ppm, measured according to ASTM D5453.

13. The hydrogenated decarboxylated rosin acid composition of any of claims 1-12, wherein the hydrogenated decarboxylated rosin acid composition has an oxidative stability measured in terms of:

   a T onset of > 168°C; and
   a T max of > 183°C, both are measured by differential scanning calorimetry (DSC).

14. A method of preparation of a hydrogenated decarboxylated rosin acid composition comprising:

   providing a decarboxylated rosin acid feedstock;
   contacting the decarboxylated rosin acid feedstock with a catalyst, optionally in the presence of a solvent, to obtain a reaction mixture;
   supplying hydrogen gas with a pressure of 500 to 2000 kPa and heating the reaction mixture at a temperature of 150°C to 330°C; and
   removing the catalyst and recovering the hydrogenated decarboxylated rosin acid composition;
   wherein the hydrogenated decarboxylated rosin acid composition comprises, based on total weight of the composition, a plurality of hydrogenated decarboxylated rosin acid (HDCR) components;
   wherein:
   45 to 100 wt.% of the HDCR components have 19 carbon atoms ($C_{19}$) and a molecular weight of 262 g/mol;

   up to 50 wt.% of the HDCR components have 19 carbon atoms ($C_{19}$) and a molecular weight of 256 g/mol;
   up to 20 wt.% of the HDCR components have 19 carbon atoms ($C_{19}$) and a molecular weight of 252 g/mol, or 19 carbon atoms ($C_{19}$) and a molecular weight of 260 g/mol; and
   up to 25 wt.% of the HDCR components have 13 carbon atoms ($C_{13}$) and a molecular weight of 180 g/mol; and

wherein the hydrogenated decarboxylated rosin acid composition has an oxygen content of < 0.5%.

15. The method of claim 14, wherein each HDCR component in the composition has a double bond equivalent (DBE); and wherein the hydrogenated decarboxylated rosin acid composition has an average DBE of 0.1 to 2, the average DBE is computed as sum of weight percent of each HDCR component multiplied by its DBE.

Europäisches
Patentamt

European
Patent Office

Office européen
des brevets

# EUROPEAN SEARCH REPORT

**Application Number**

EP 24 20 0489

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | EP 4 339 265 A1 (KRATON POLYMERS NEDERLAND B V [NL]) 20 March 2024 (2024-03-20) * claims 8-9 * ----- | 1-15 | INV. C08L93/04 |
| | | | TECHNICAL FIELDS SEARCHED (IPC) |
| | | | C08L |

The present search report has been drawn up for all claims

| Place of search | Date of completion of the search | Examiner |
|---|---|---|
| Munich | 29 January 2025 | Pellegrini, Paolo |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 20 0489

This annex lists the patent family members relating to the patent documents cited in the above-mentioned European search report.
The members are as contained in the European Patent Office EDP file on
The European Patent Office is in no way liable for these particulars which are merely given for the purpose of information.

29-01-2025

| Patent document cited in search report | | Publication date | Patent family member(s) | | Publication date |
|---|---|---|---|---|---|
| EP 4339265 | A1 | 20-03-2024 | EP | 4339265 A1 | 20-03-2024 |
| | | | US | 2024093118 A1 | 21-03-2024 |

EPO FORM P0459

For more details about this annex : see Official Journal of the European Patent Office, No. 12/82